# EUROPEAN PATENT APPLICATION

(11) **EP 2 149 790 A1**
(43) Date of publication of application: **03.02.2010**
(21) Application number: 08740307.7
(22) Date of filing: 12.04.2008
(51) Int. Cl.: G01N 27/28, A61B 5/157, G01N 27/327, G01N 27/416

(54) **ANALYZING TOOL**

(30) Priority: 12.04.2007 JP 2007105292
(71) Applicant: Arkray, Inc., Kyoto-shi, Kyoto 601-8045 (JP)
(72) Inventor: KUSAKA, Yasuhide, Kyoto-shi Kyoto 601-8045 (JP); KIMURA, Sadaaki, Kyoto-shi Kyoto 601-8045 (JP)
(74) Representative: HOFFMANN EITLE
(86) International application number: PCT/JP2008/057218
(87) International publication number: WO 2008/126923

(57) **Abstract**

The present invention relates to an analytical instrument including: a plurality of electrodes 10, 11 and 12 formed in an annular shape with stacked on one another in an axial direction; and a specimen layer formed in an interior of at least one of the plurality of electrodes 10, 11 and 12. The analytical instrument according to the present invention may be configured to include: a first electrode formed in an annular shape; a second electrode formed in an annular shape and inserted through an interior of the first electrode with a space from an inner peripheral surface of the first electrode; and a specimen layer formed between the inner peripheral surface of the first electrode and an outer peripheral surface of the second electrode.

## Description

### TECHNICAL FIELD

The present invention relates to an analytical instrument used to analyze a particular component (for example, glucose, cholesterol or lactic acid) in a specimen (for example, biochemical specimen such as blood or urine).

### BACKGROUND TECHNOLOGY

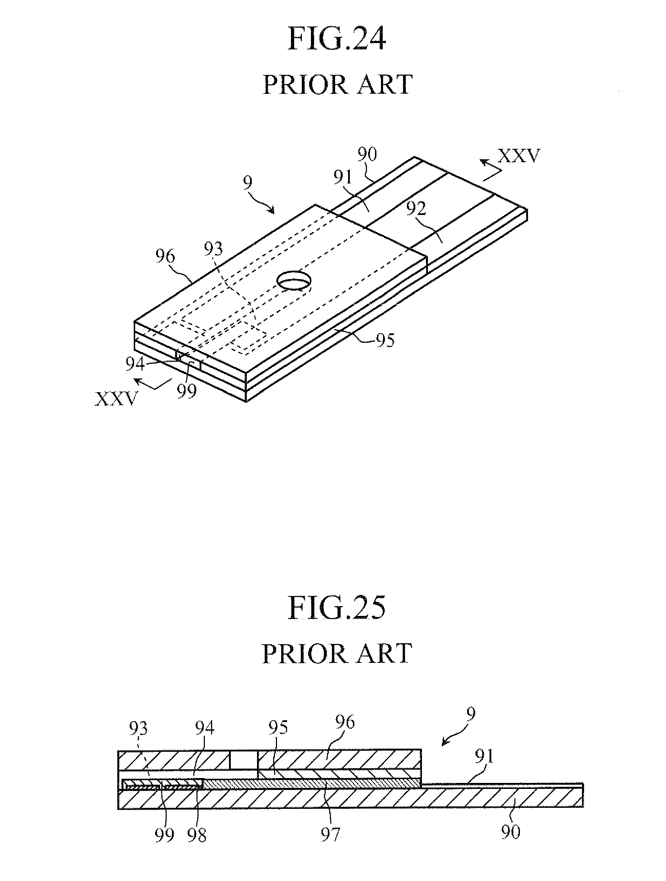

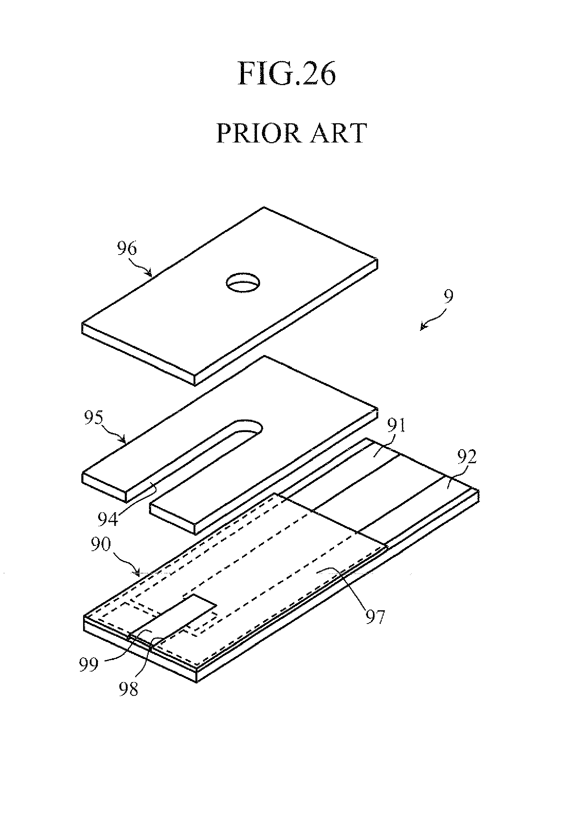

In the conventional methods for measuring a glucose concentration in blood, a disposable analytical instrument is often used for easy handling (for example, see the Patent Document 1). An example of the analytical instrument is adapted to measure a response current value necessary for the calculation of a blood glucose level using a working electrode 91 and a counter electrode 92 provided on a substrate 90 in a manner similar to a glucose sensor 9 illustrated in Figs. 24 to 26. The glucose sensor 9 transfers blood using a capillary force generated in a capillary 93 and measures an amount of electrons transferred when the glucose in blood and the specimen are reacted with each other as the response current value in the working electrode 91. The capillary 93 is formed by overlaying a cover 96 on the substrate 90 with a spacer 95 provided with a slit 94 interposed therebetween. The substrate 90 retains the specimen in a specimen portion 99 provided in an opening 98 of an insulation film 97.

[Patent Document 1] Japanese Patent Publication H08-10208

### DISCLOSURE OF THE INVENTION

### PROBLEMS TO BE SOLVED BY THE INVENTION

A sensitivity of the glucose sensor 9 is variable depending on an area on a part of the working electrode 91 exposed in the capillary 93. On the contrary, it is difficult to secure a large area for the working electrode 91 in a structure wherein the cover 96 overlays the substrate 90 with the spacer 95 interposed therebetween and the working electrode 91 is provided on the substrate 90. In order to reduce an amount of blood sampling, it is necessary to downsize the glucose sensor 9 so that the capillary has a smaller volume, nevertheless, a higher sensitivity is demanded. All these facts inevitably make it difficult to ensure a high sensitivity and also meet the demand for downsizing in the glucose sensor 9.

A main object of the present invention is to provide an analytical instrument capable of ensuring a high sensitivity while meeting the demand for downsizing.

### MEANS FOR SOLVING THE PROBLEM

A first aspect of the present invention provides an analytical instrument including: a plurality of electrodes formed in an annular shape with stacked on each other in an axial direction; and a specimen layer formed in an interior of at least one of the plurality of electrodes.

An insulation layer, for example, is interposed between the adjacent electrodes in the plurality of electrode. The insulation layer preferably joins the adjacent electrodes with each other.

The adjacent electrodes in the plurality of electrode may be coupled with each other by an insulation tube provided to mantle these electrodes with a certain space therebetween. The insulation tube is preferably a heat-shrinkable tube.

The analytical instrument according to the present invention may further include a sealing member for embracing outer peripheral surfaces of the plurality of electrodes. The sealing member preferably has through holes for exposing part of the outer peripheral surfaces of the plurality of electrodes.

The plurality of electrodes include, for example, a working electrode and a counter electrode. The plurality of electrodes may further include a detection electrode for detecting the supply of a specimen to the interiors of these electrodes.

The interiors of the plurality of electrodes are preferably configured to exert a capillary force.

An insulation layer may be provided on an end surface of an outermost one in the plurality of electrodes.

A second aspect of the present invention provides an analytical instrument including: a first electrode formed in an annular shape; a second electrode formed in an annular shape and inserted through an interior of the first electrode with a space from an inner peripheral surface of the first electrode; and a specimen layer formed between the inner peripheral surface of the first electrode and an outer peripheral surface of the second electrode.

For example, an end section of the second electrode projects from an end surface of the first electrode.

For example, an end section of the second electrode is retracted behind an end surface of the first electrode.

Preferably, an end section of the second electrode projects from an end surface of the first electrode, and another end section of the second electrode is retracted behind another end surface of the first electrode.

The first electrode and the second electrode are joined with each other by, for example, an adhesive. The adhesive is preferably supplied into through holes formed in the first electrode and further adherently extended to an outer peripheral surface of the second electrode.

Preferably, the first electrode is a working electrode, and the second electrode is a counter electrode, in which case the specimen layer is preferably formed on an inner peripheral surface of the first electrode.

A space between the inner peripheral surface of the first electrode and the outer peripheral surface of the second electrode is, for example, configured to exert a capillary force.

At least one of an end surface and an inner surface in a lower end section of the second electrode may be hydrophobized.

### BRIEF DESCRIPTION OF THE DRAWINGS

Fig. 1 is an overall perspective view of a biosensor according to a preferred embodiment 1 of the present invention.
Fig. 2 is a sectional view cut along II-II Line illustrated in Fig. 1.
Fig. 3 is an overall perspective view illustrating another example of the biosensor.
Fig. 4 is a perspective view for describing a method for manufacturing the biosensor illustrated in Fig. 1.
Fig. 5 is a perspective view for describing the method for manufacturing the biosensor illustrated in Fig. 1.
Fig. 6 is a perspective view for describing the method for manufacturing the biosensor illustrated in Fig. 1.
Fig. 7 is a perspective view for describing the method for manufacturing the biosensor illustrated in Fig. 1.
Fig. 8 is a perspective view for describing the method for manufacturing the biosensor illustrated in Fig. 1.
Fig. 9 is a perspective view for describing the method for manufacturing the biosensor illustrated in Fig. 1.
Fig. 10 is an overall perspective view of a biosensor according to a preferred embodiment 2 of the present invention.
Fig. 11 is a sectional view cut along XI-VI Line illustrated in Fig. 10.
Fig. 12 is a sectional view cut along XII-XII Line illustrated in Fig. 10.
Fig. 13 is an overall perspective view of a biosensor according to a preferred embodiment 3 of the present invention.
Fig. 14 is a sectional view cut along XIV-XIV Line illustrated in Fig. 13.
Fig. 15 is an overall perspective view of a biosensor according to a preferred embodiment 4 of the present invention.
Fig. 16 is a sectional view cut along XVI-XVI Line illustrated in Fig. 15.
Figs. 17A and 17B are perspective views for describing a method for manufacturing the biosensor illustrated in Fig. 15.
Fig. 18 is a perspective view for describing the method for manufacturing the biosensor illustrated in Fig. 15.
Fig. 19 is an overall perspective view illustrating a device according to a preferred embodiment 5 of the present invention by removing a part of the device.
Fig. 20 is a sectional view cut along XX-XX Line illustrated in Fig. 19.
Fig. 21 is a sectional view illustrating a state where the device of Fig. 19 is mounted in a puncture device.
Figs. 22A and 22B are sectional views for describing operations of the device illustrated in Fig. 19 and the puncture device illustrated in Fig. 21.
Fig. 23 is a sectional view for describing the operations of the device illustrated in Fig. 19 and the puncture device illustrated in Fig. 21.
Fig. 24 is an overall perspective view illustrating a conventional biosensor.
Fig. 25 is a sectional view cut along XXV-XXV Line illustrated in Fig. 24.
Fig. 26 is an exploded perspective view of the biosensor illustrated in Fig. 24.

### DESCRIPTION OF REFERENCE SYMBOLS

- 1, 1': biosensor (analytical instrument)
- 10: working electrode
- 11: counter electrode
- 12: detection electrode
- 14: specimen layer
- 15, 16: insulation layer
- 3: biosensor
- 31: sealing member
- 32, 33, 34: contact hole (through hole)
- 4: biosensor (analytical instrument)
- 42, 43: (insulation) tube
- 5: biosensor
- 50: working electrode (first electrode)
- 50b: end surface (one end surface) (of working electrode)
- 50c: end surface (another end surface) (of working electrode)
- 51: counter electrode (second electrode)
- 51a: outer peripheral surface (of counter electrode)
- 52: specimen layer
- 53: through hole
- 54: adhesive
- 56: upper end section (one end section) (of counter electrode)
- 57: lower end section (another end section) (of counter electrode)

### PREFERRED EMBODIMENT OF THE INVENTION

Hereinafter, preferred embodiments 1 to 5 of the present invention are described referring to the drawings.

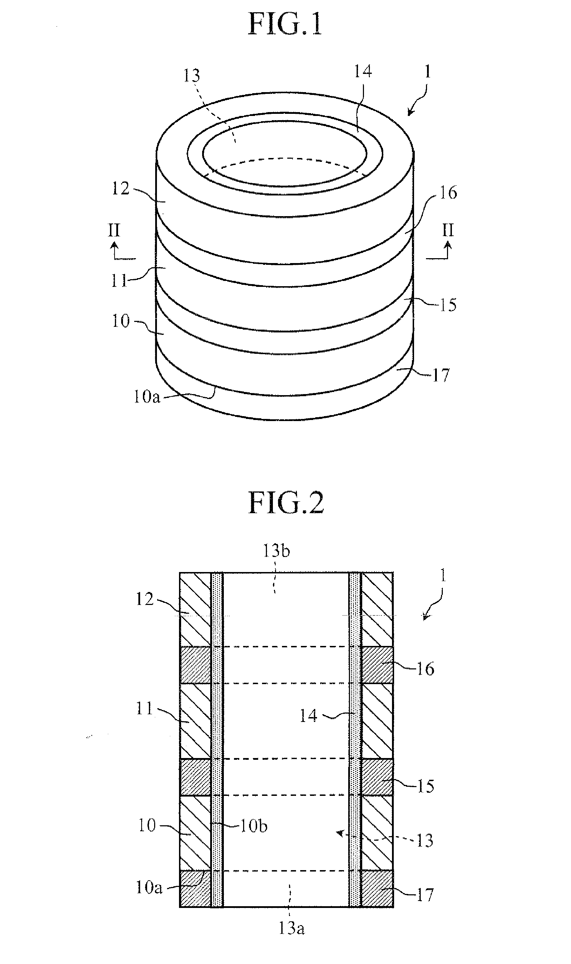

First, the preferred embodiment 1 is described referring to Figs. 1 to 9.

A biosensor 1 illustrated in Figs. 1 and 2 is configured to be disposable, and an overall shape thereof is a cylindrical shape. The biosensor 1 is loaded in a device (not shown) having an analyzing function such as a concentration measurement device to be used to analyze a particular component (for example, glucose, cholesterol or lactic acid) in a specimen (for example, biochemical specimen such as blood or urine). The biosensor 1 includes a working electrode 10, a counter electrode 11, a detection electrode 12, a capillary 13, and a specimen layer 14.

The working electrode 10 and the counter electrode 11 apply a voltage to the specimen introduced into the capillary 13 and also used to measure a response current generated then. The detection electrode 12 is used to detect whether or not the capillary 13 has been supplied with a predetermined amount of specimen.

The working electrode 10, the counter electrode 11 and the detection electrode 12 are formed in an annular shape having equal or substantially equal outer and inner diameters. The working electrode 10, the counter electrode 11 and the detection electrode 12 are stacked on one another in an axial direction in this order so that axial centers of these electrodes coincide or substantially coincide with one another. The working electrode 10, the counter electrode 11 and the detection electrode 12 are made of, for example, nickel, iron, copper, alloy obtained from these metals, stainless steel or carbon, and formed so as to have an outer diameter of 0.5 to 2.0 mm, an inner diameter of 0.2 to 1.6 mm, and an axial dimension of 1.0 to 5.0 mm.

The working electrode 10, the counter electrode 11 and the detection electrode 12 are not necessarily provided in the order illustrated in the drawing but may be differently ordered. The detection electrode 12 may be omitted, and two electrodes, that are the working electrode 10 and the counter electrode 11, alone may be used.

The working electrode 10 and the counter electrode 11, and the counter electrode 11 and the detection electrode 12 are joined with each other with adhesive layers 15 and 16 having insulation properties, respectively. The adhesive layers 15 and 16 may be wholly made of an adhesive material, or may be a medium provided with the adhesive material on both surfaces thereof. Examples of the adhesive material usable for the adhesive layers 15 and 16 are synthetic adhesives such as: thermosetting resin (for example, urea resin, melamine resin, resorcinol resin, phenol resin, epoxy resin, polyurethane resin, polyaromatic resin or polyester resin); and thermoplastic resin (for example, vinyl acetate resin, polyvinylalcohol resin, polyvinylacetal resin, polyvinyl resin, acrylic resin, polyethylene resin or cellulose resin); elastomeric adhesive (chloroprene rubber adhesive, nitrile rubber adhesive, SBR adhesive, SBR-SIS adhesive, polysulfide rubber adhesive, butyl rubber adhesive, or silicone rubber adhesive). A particularly preferable example of the adhesive is an epoxy resin adhesive superior in its adhesive strength such as EPOXY REIN XNR 3506 (manufactured by Nagase ChemteX Corporation) OR EPOXY REXIN XNR 3501 (manufactured by Nagase ChemteX Corporation).

An insulation layer 17 is formed on an end surface 10a of the working electrode 10. The insulation layer 17 is provided to prevent the specimen from sticking to an outer peripheral surface of the working electrode 10. A structure of and a material for the insulation layer 17 may be similar to those of the adhesive layers 15 and 16.

The capillary 13 transfers the specimen introduced through an opening 13a toward an opening 13b utilizing capillarity and then retains the introduced specimen. The capillarity 13 is defined by inner surfaces of the working electrode 10, counter electrode 11, detection electrode 12 and adhesive layers 15 and 16, and its volume is set to, for example, 0.03 to 10 µL.

The specimen layer 14 is provided so as to bridge the working electrode 10, the counter electrode 10 and the detection electrode 12 in the capillary 13. The specimen layer 14 includes an electron transmitter and oxidoreductase and is formed in such a solid shape that is easily dissolved when making contact with the specimen. Therefore, when the specimen is introduced into the capillary 13, the specimen layer 14 is dissolved, and a liquid phase reaction system including the electron transmitter, oxidoreductase and specimen is built in the capillary 13.

The selection of the oxidoreductase depends on the type of the particular component to be analyzed. When the glucose, for example, is analyzed, glucose hydrogenase (GDH) or glucose oxidase (GOD) can be used. Examples of the electron transmitter are ruthenium complex and iron complex, and [Ru(NH₃)₆]Cl₃ or K₃[Fe(CN)₆] can be typically used.

The specimen layer 14 is provided so as to bridge the working electrode 10, the counter electrode 11 and the detection electrode 12 in the illustrated example. The specimen layer 14 may be differently formed as far as at least the inner surface of the working electrode 10 can be thereby covered.

In the biosensor 1, the working electrode 10 is cylindrically formed, electrons can be transferred to and from the electron transmitter on an entire area of an inner surface 10b thereof. Accordingly, the biosensor 1 can reliably acquire a larger electron receiving surface (inner surface 10b) in the working electrode 10 than in the conventional biosensor 9 having a plate shape wherein the working electrode 91 is formed in a shape of a film on the substrate 90 (see Figs. 24 to 26). Further, a planar dimension of the electron receiving surface (inner surface 10b) in the working electrode 10 can be easily adjusted according to an inner diameter and a height dimension of the working electrode. In a case where the biosensor 1 is downsized, therefore, the electron receiving surface (inner surface 10b) of the working electrode 10 per unit volume of the capillary 13 can be fairly large when the inner diameter and the height dimension of the working electrode 10 are suitably set. As a result, the biosensor 1, though downsized, can reliably achieve a high sensitivity.

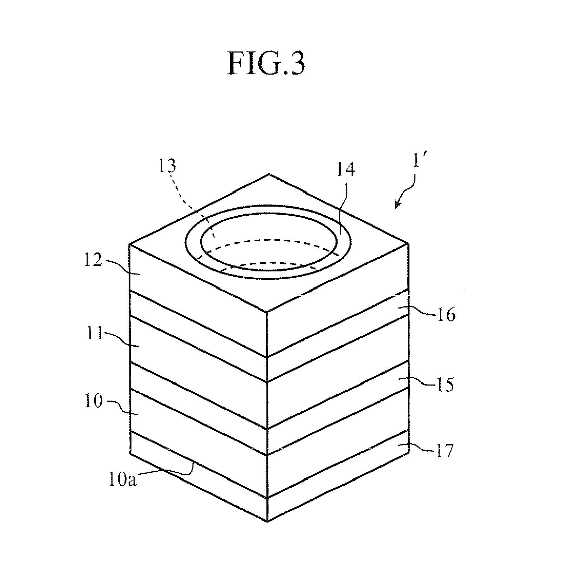

The biosensor I illustrated in Figs. 1 and 2 is overall cylindrically shaped. The shape, however, may be a cube shape as in a biosensor 1' illustrated in Fig. 3. In Fig. 3, any structural elements similar to those of the biosensor 1 illustrated in Figs. 1 and 2 are shown with the same reference symbols attached thereto.

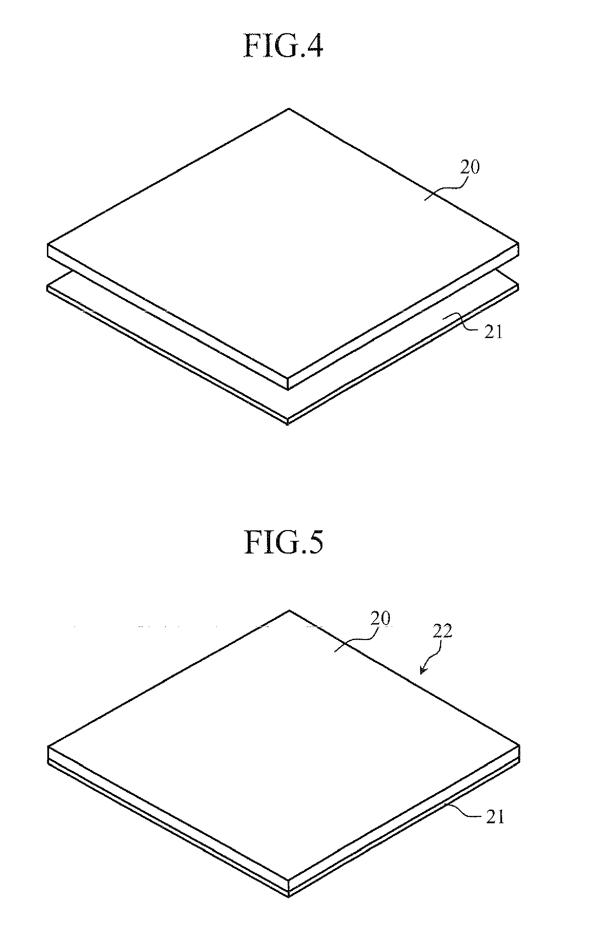

Next, a method for manufacturing the biosensor 1 is described referring to Figs. 4 to 9.

As illustrated in Figs. 4 and 5, a double-stick adhesive sheet 21 having insulation properties is bonded to a metal plate 20 so that a laminated plate 22 is formed. An example of the metal plate 20 is a plate made of nickel, iron, copper, alloys obtained from these metals, stainless steel or carbon and formed in a thickness of 0.1 to 2.0 mm. An example of the double-stick adhesive sheet 21 is a sheet having a thickness of 0.015 to 0.5 mm obtained by forming an adhesive layer made of acrylic resin, silicone resin or the like on both surfaces of a medium such as a polyester film. As the double-stick adhesive sheet 21 may be used a simple adhesive layer in which no medium is used.

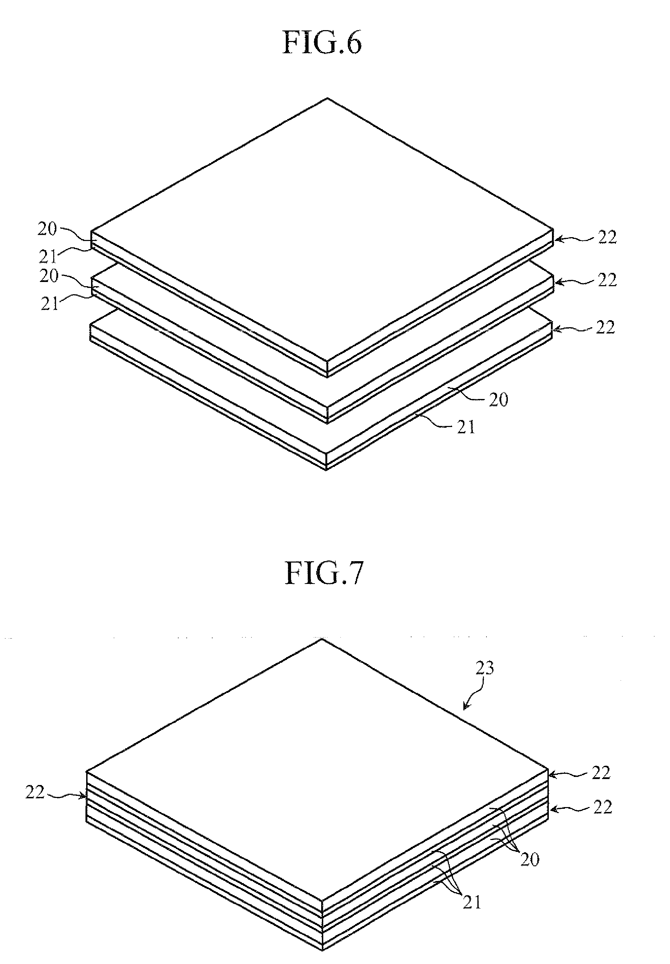

As illustrated in Figs. 6 and 7, three laminated plates 22 are bonded to one another so that the respective double-stick adhesive sheets 21 are sandwiched with the metal plates 20 interposed therebetween, so that a multilayered product 23 is formed.

In a case where the detection electrode 12 is omitted in the biosensor 1, two laminated plates 22 are bonded to each other to constitute the multilayered product 23.

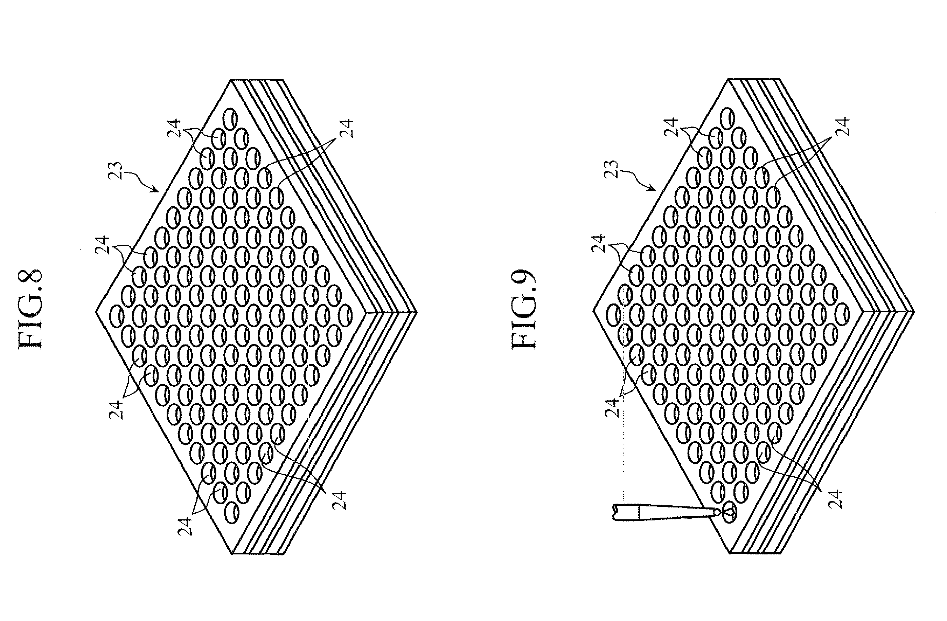

Then, a plurality of through holes 24 are formed in a matrix shape in the multilayered product 23 as illustrated in Fig. 8. The through hole 24 is formed by drilling, punching or laser processing in a columnar shape having a diameter of 0.3 to 2.0 mm and a volume of 0.05 to 10 µL.

As illustrated in Fig. 9, a specimen-containing agent in liquid or slurry state is applied to the plurality of through holes 24. After the specimen-containing agent is applied to the through holes 24, the specimen-containing agent is sucked into the through holes 24 by the capillarity generated in the through holes 24. A conventional dispenser can be used for the application of the specimen-containing agent. The specimen-containing agent includes, for example, oxidoreductase suitable for the particular component to be analyzed and the electron transmitter such as ruthenium complex or iron complex.

When interiors of the plurality of through holes 24 are filled with the specimen-containing agent, moisture in the specimen-containing agent is evaporated by heating or the like, and the specimen in solid state is thereby attached to inner surfaces of the through holes 24 (see Fig. 2). In a case where the specimen-containing agent includes oxidoreductase, it is necessary to heat the specimen-containing agent in such a manner that the enzyme is neither deactivated nor modified.

Finally, the multilayered product 23 is subject to die-cutting on the periphery of the through hole 24. As a result, the cylindrical biosensor 1 illustrated in Figs. 1 and 2 can be obtained. When the multilayered product 23 is cut longitudinally and laterally between the plurality of through holes 23, the cube-shape biosensor 1' illustrated in Fig. 3 can be obtained.

As described, the biosensors 1 and 1' are capable of ensuring a high sensitivity while meeting the demand for downsizing, and can be advantageously manufactured in such a simplified process.

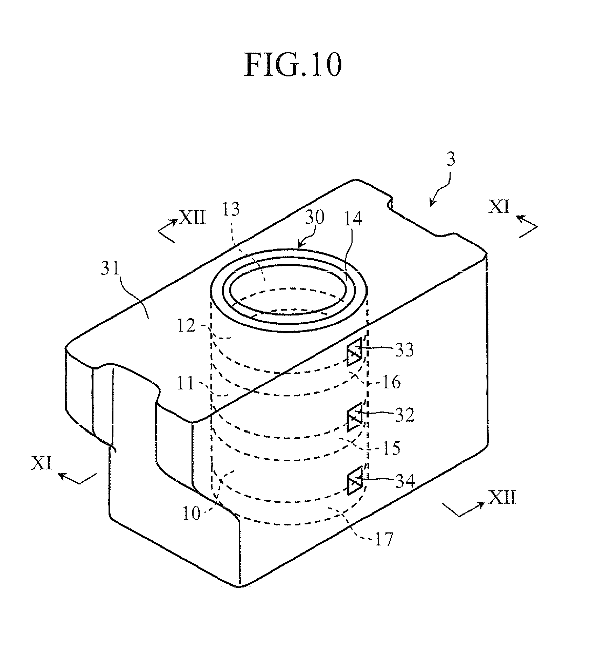

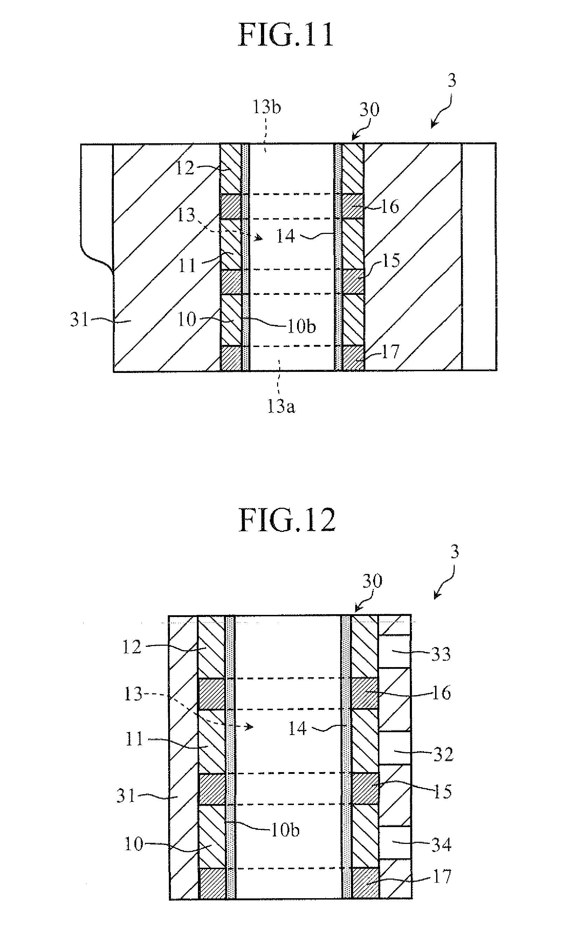

A preferred embodiment 2 of the present invention is described below referring to Figs. 10 to 12. In Figs. 10 to 12, any structural elements similar to those of the biosensor 1 described earlier referring to Figs. 1 and 2 are shown with the same reference symbols attached thereto, and the description of these structural elements will not be given.

A biosensor 3 illustrated in Figs. 10 to 12 includes a sensor body 30 and a sealing member 31.

The sensor body 30 corresponds to the biosensor 1 illustrated in Figs. 1 and 2.

The sealing member 31 protects the sensor body 30 and also improves handle-ability of the biosensor 3. The sealing member 31 is formed by resin molding using thermoplastic resin such as acryl, nylon or polyacetate or thermosetting resin such as epoxy resin or polyester resin so as to embrace an outer peripheral surface of the sensor body 30. The sealing member 31 is provided with three contact holes 32, 33 and 34. The contact holes 32 to 34 are provided to allow an external connector (not shown) to be brought in contact with the working electrode 10, the counting electrode 11 and the detection electrode 12 in the sensor body 30. The contact holes 32 to 34 are formed so as to expose part of the outer peripheral surfaces of the working electrode 10, the counter electrode 11 and the detection electrode 12.

The handle-ability of the biosensor 3 thus constituted can be improved by the sealing member 31 as described earlier. The biosensor 3 is further advantageous in a case where a plurality of biosensors 3 are aligned and housed in the form of a cartridge.

In the biosensor 3, the device body 30 corresponding to the biosensor 1 illustrated in Figs. 1 and 2 is used, so that a manufacturing process can be simplified and a high sensitivity can be reliably attained.

It is needless to say that the cube-shape biosensor 1' illustrated in Fig. 3 can be used as the sensor body 30.

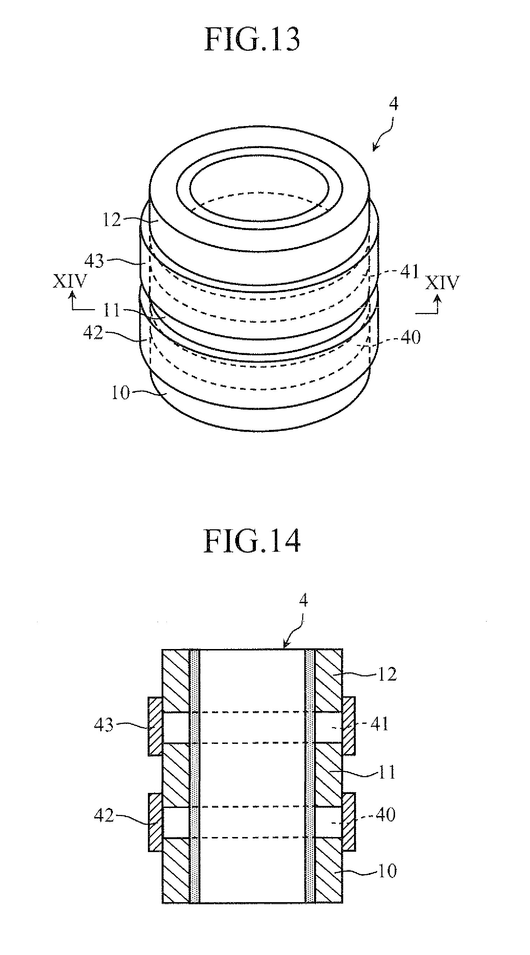

A preferred embodiment 3 of the present invention is described below referring to Figs. 13 and 14. In Figs. 13 and 14, any structural elements similar to those of the biosensor 1 described earlier referring to Figs. 1 and 2 are shown with the same reference symbols attached thereto, and the description of these structural elements will not be given.

A biosensor 4 illustrated in Figs. 13 and 14 includes a working electrode 10, a counter electrode 11 and a detection electrode 12. These electrodes are coupled with each other in an axial direction by tubes 42 and 43, respectively, with spaces 40 and 41 provided therebetween.

The tubes 42 and 43 have insulation properties. The tubers 42 and 43 couple the two adjacent electrodes 10 and 11 (11 and 12) with each other in a state where they mantle the electrodes 10 and 11 (11 and 12). The electrodes 10 to 12 are electrically isolated from one another because the tubes 42 and 43 have insulation properties and the spaces 40 and 41 are provided between the respective electrodes 10 to 12.

A heat-shrinkable product can be used as the tubes 42 and 43. The heat-shrinkable tubes 42 and 43 can be made of, for example, silicone rubber, ethylene propylene rubber, polyvinyl chloride, elastic neoprene, polyimide or fluororesin).

When the electrodes 10 and 11 (11 and 12) are coupled with each other by the heat-shrinkable tubes 42, 43, the electrodes 10 and 11 (11 and 12) are placed on each other with the space 40, 41 interposed therebetween, and the tubes 42, 43 are heated to be shrunk after the adjacent electrodes 10 and 11 (11 and 12) are mantled by the tubes 42, 43. The coupling method thus constituted can be very easily performed, which advantageously facilitates the manufacturing of the biosensor 4.

The working electrode 10, the counter electrode 11 and the detection electrode 12 are not necessarily provided in the biosensor 4 in the order illustrated in the drawing but may be differently ordered. The detection electrode 12 may be omitted, and two electrodes, that are the working electrode and the counter electrode 11, alone may be used.

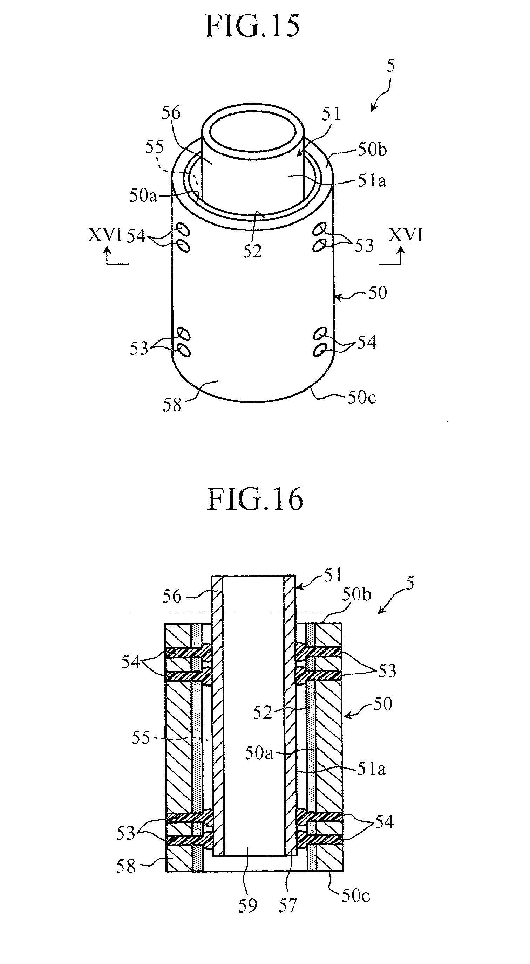

Next, a preferred embodiment 4 of the present invention is described referring to Figs. 15 to 18.

A biosensor 5 illustrated in Figs. 15 and 16 includes a working electrode 50, a counter electrode 51 and a specimen layer 52.

The working electrode 50 is formed in an annular shape having an inner diameter larger than an outer diameter of the counter electrode 51, and accordingly mantles the counter electrode 51. The working electrode 50 is formed so as to have such dimensions as an outer diameter of 2.0 to 5.0 mm, an inner diameter of 1.0 to 4.5 mm, and an axial dimension of 1.0 to 5.0 mm. The working electrode 50 has a plurality of through holes 53, and supports the counter electrode 51 by means of these through holes 53. More specifically, the plurality of through holes 53 are filled with an adhesive 54 having insulation properties, and part of the adhesive 54 further extends to an outer peripheral surface 51a of the counter electrode 51, so that the counter electrode 51 is supported by the working electrode 50. An inner diameter of the through hole 53 is set to, for example, 0.1 to 1.0 mm.

The counter electrode 51 has an annular shape, and is supported by the working electrode 50 with inserted through an interior of the working electrode 50. The counter electrode 51 has such dimensions as an outer diameter of 0.8 to 4.5 mm, an inner diameter of 0.5 to 4.0 mm, and an axial dimension of 1.0 to 10 mm.

Because the working electrode 50 is formed in such an annular shape that has the inner diameter larger than the outer diameter of the counter electrode 51, a space 55 is formed between the outer peripheral surface 51a of the counter electrode 51 and an inner surface 50a of the working electrode 50. The space 55 serves as a capillary which exerts a capillary force to thereby introduce the specimen thereinto. An upper end section 56 of the counter electrode 51 projects from an end surface 50b of the working electrode 50, and a lower end section 57 thereof is retracted behind an end surface 50c of the working electrode 50. At least one of an end surface 51c and an inner surface 51d of the lower end section 57 in the counter electrode 51 may be hydrophobized. Any of the various conventional treatments can be employed to hydrophobize these surfaces, for example, the surfaces can be coated with hydrophobic resin such as a fluorine compound.

The specimen layer 52 is constituted in a manner similar to the specimen layer 14 in the biosensor 1 descried earlier (see Fig. 2). The specimen layer 52 is formed on the inner surface 50a of the working electrode 50 to be present in the space 55. The space 55 is used to introduce the specimen as described earlier, therefore, the specimen layer 52 is dissolved by the specimen introduced into the space 55.

As far as the specimen layer 52 is present in the space 55, the location of the specimen layer 52 is not necessarily limited to the inner surface 50a of the working electrode 50 but may be the outer peripheral surface 51a of the counter electrode 51.

In the biosensor 5, the working electrode 50 is cylindrically formed, electrons can be transferred to and from the electron transmitter in an entire area of the inner surface 50a thereof. Accordingly, the biosensor 1 can reliably acquire a larger electron receiving surface (inner surface 50a) in the working electrode 50 than in the conventional biosensor 9 having a plate shape wherein the working electrode 91 is formed in a shape of a film on the substrate 90 (see Figs. 24 to 26). Further, with the working electrode 50 mantling the counter electrode 51, the electron receiving surface (inner surface 50a) can be further increased as compared with a structure wherein the working electrode 50 and the counter electrode 51 are axially coupled with each other. In a case where the biosensor 1 is downsized, the electron receiving surface (inner surface 50a) of the working electrode 50 per unit volume of the capillary (space 55) can still be fairly large. As a result, the biosensor 5, though downsized, can ensure a high sensitivity.

In the biosensor 5, the upper end section 56 of the counter electrode 51 projects from the end surface 50b of the working electrode 50, a contact point between an external connector (not shown) and the counter electrode 51 can be easily obtained. Further, in the biosensor 5, the lower end section 57 of the counter electrode 51 is retracted behind the end surface 50c of the working electrode 50, the specimen can be prevented from entering an interior 59 of the counter electrode 51 when the specimen is attached to the lower end section 58 of the biosensor 5. When the end surface 51c of the counter electrode 51 and the inner surface 51d of the lower end section 57 are hydrophobized, the specimen can be more effectively prevented from entering the interior 58 of the counter electrode 51.

Next, a method for manufacturing the biosensor 5 is described.

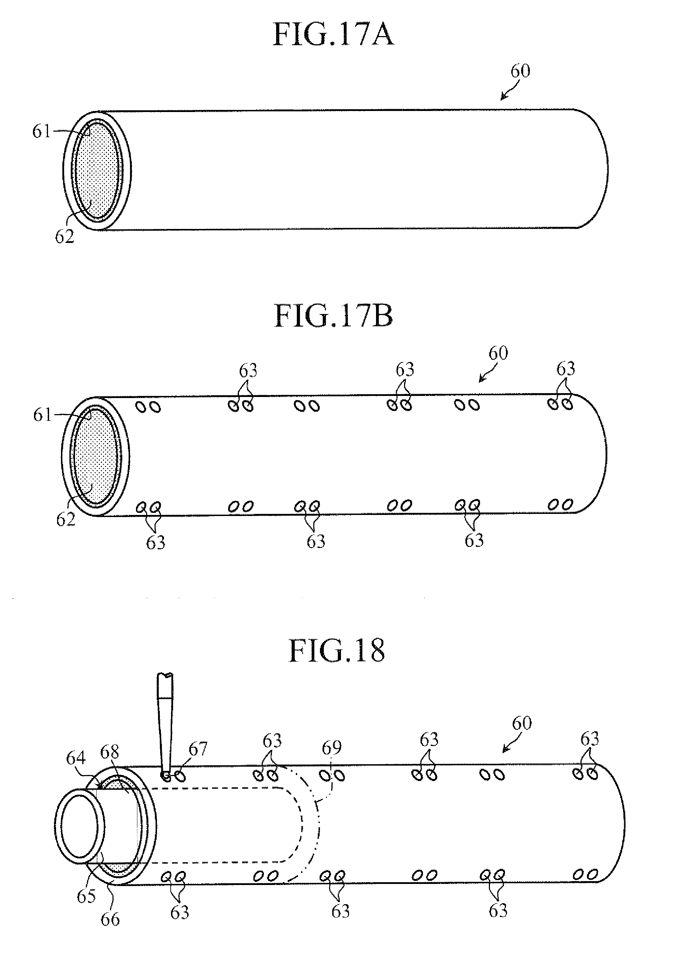

First, a specimen layer 62 is formed on an inner surface 61 of a tube 60 having a large diameter as illustrated in Fig. 17A. The specimen layer 62 can be formed by filling an interior of the tube 60 with the specimen-containing agent and then evaporating moisture in the specimen-containing agent.

Next, a plurality of through holes 63 are formed at predetermined positions in the tube 60 as illustrated in Fig. 17B. The through holes 63 are formed by laser processing or die-cutting.

Then, a tube 64 having a small diameter and a predetermined length is inserted through the interior of the tube 60 as illustrated in Fig. 18. The tube 64 is inserted so that an end section 65 of the tube 64 projects from an end surface 66 of the tube 64. An adhesive 67, such as resin, is supplied into the through holes 63 of the tube 60. An amount of the adhesive 67 to be supplied is preferably larger than a volume of the through hole 63 because, accordingly, the adhesive 67 can fill the through holes while being attached to an outer surface 68 of the tube 64. After the supply of the adhesive 67 to the through holes 63, the adhesive 67 is cured.

Finally, the tubes 60 and 64 are cut along a cutting plane line 69 shown with a dotted line in Fig. 18 after the adhesive 67 is cured. As a result, the biosensor 5 illustrated in Fig. 15 and 16 can be obtained. The cutting plane line 69 is set so that the lower end section 57 of the counter electrode 51 can be retracted behind the end surface50c of the working electrode 50 (see Fig. 16).

The biosensor 5 thus constituted makes it unnecessary to form electrodes by, for example, screen printing, and can be thereby easily and inexpensively manufactured.

In the biosensor 5, it is a selectable matter whether the upper end section 56 of the counter electrode 51 projects from the end surface 50b of the working electrode 50 or the lower end section 57 of the counter electrode 51 is retracted behind the end surface 50c of the working electrode 50. Further, the inner tube may be used as a working electrode, and the outer tube may be used as a counter electrode. The biosensor 5 may have its periphery sealed with, for example, resin in the same manner as the biosensor 3 described referring to Figs. 10 to 12.

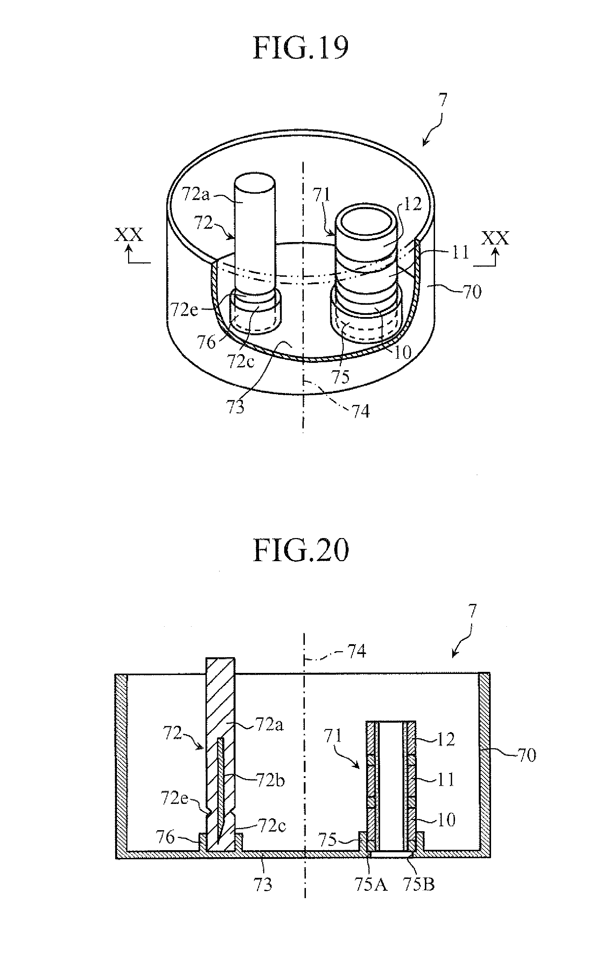

Next, a preferred embodiment 5 of the present invention is described referring to Figs. 19 to 23. In Figs. 19 to 23, any structural elements similar to those of the biosensor 1 described earlier referring to Figs. 1 and 2 are shown with the same reference symbols attached thereto, and the description of these structural elements will not be given.

A device 7 illustrated in Figs. 19 and 20 retains a biosensor 71 and a lancet 72 in a case 70.

The case 70 is formed in a cylindrical shape in which an upper portion is open, and a lower part is provided with a bottom wall 73. The case 70 includes two retainers 75 and 76 provided at positions offset by a substantially equal distance from a center 74 of the bottom wall 73. The retainer 75 is provided to retain the biosensor 71, while the retainer 76 is provided to retain the lancet 72. A bottom section 75A of the retainer 75 is provided with a through hole 75B. The through hole 75B allows a puncture needle 72b of the lancet 72, described later, to pass therethrough, and communicates with an interior of the biosensor 71.

The biosensor 71 is constituted in a manner similar to the biosensor 1 described earlier referring to Figs. 1 and 2, and retained in the retainer 75 so that the working electrode 10 is located downside. As a matter of course, the biosensor 1' described referring to Fig. 3 and the biosensor 5 described referring to Figs. 15 and 16 can be used as the biosensor 71.

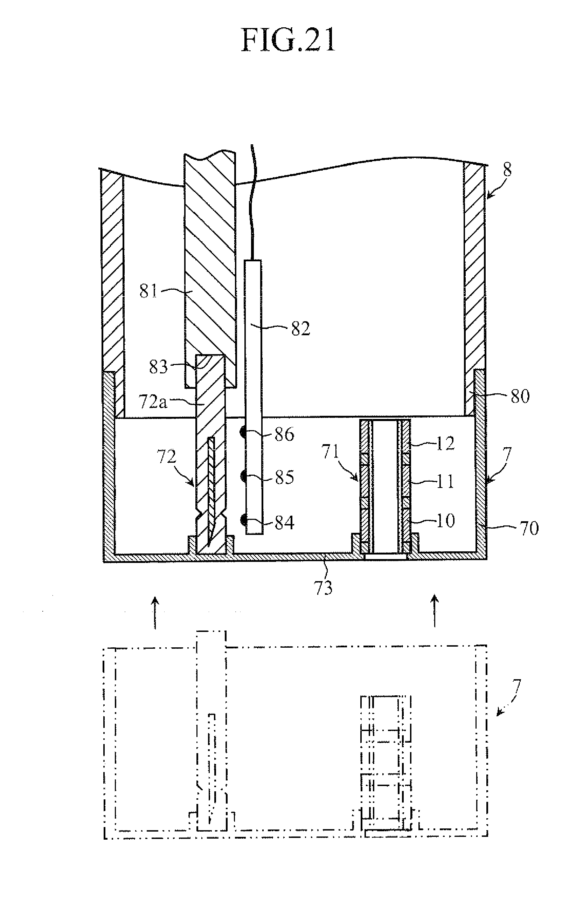

The lancet 72 is provided to incise a body part to be punctured such as skin, and includes a columnar portion 72a, a puncture needle 72b and a cap 72c. The columnar portion 72a is mounted in a lancet holder 81 (see Fig. 21) of a puncture device 8 described later, and serves to retain the puncture needle 72b. The puncture needle 72b is punctured into skin, and a top section 72d thereof projects from the columnar portion 72a. The cap 72c covers the top section 72d of the puncture needle 72b. A fragile portion 72e is provided between the cap 72c and the columnar portion 72a. The fragile portion 72e is formed in the lancet 72 to make it easier to wring the cap 72c off. In resin molding, the fragile portion 72e is formed between the columnar portion 72a and the cap 72c, and the puncture needle 72b is inserted therethrough, so that the lancet 72 is obtained.

As illustrated in Fig. 21, the device 7 is mounted at a top section 80 of a puncture device 8 to be used. The puncture device 8 includes a lancet holder 81 and a connector 82.

The lancet holder 81 includes a recessed portion 83 for retaining the columnar portion 72a of the lancet 72, and is allowed to reciprocate upward and downward by a drive mechanism not shown. Examples of the drive mechanism are a latch mechanism in which springs are used, and a mechanism utilizing a motor rotational force, magnetic force or electromagnetic force.

The connector 82 applies a voltage to between the working electrode 10 and the counter electrode 11, or between the working electrode 10 (counter electrode 11) and the detection electrode 12, and measures a current between the electrodes 10 to 12. The connector 82 includes contact points 84, 85 and 86 to be brought in contact with the working electrode 10, the counter electrode 11 and the detection electrode 12. The contact points 84 to 86 are provided on a movement locus of the biosensor 7 when the case 70 is rotated on the center of the bottom wall 73.

Next are described puncture and analysis operations in which the device 7 and the puncture device 8 are used.

First, the device 7 is loaded in the puncture device 8 in a state where the lancet 72 is position-adjusted to the lancet holder 81 as illustrated in Fig. 21. Accordingly, the columnar portion 72a of the lancet 72 is fitted into the recessed portion 82 of the lancet holder 81. When the lancet 72 is position-adjusted to the lancet holder 81, for example, a protruding portion and a recessed portion to be meshed with each other are provided at end sections of the case 70 and the puncture device 8, and the device 7 is loaded in the puncture device 8 so that the protruding portions and the recessed portions are meshed with each other.

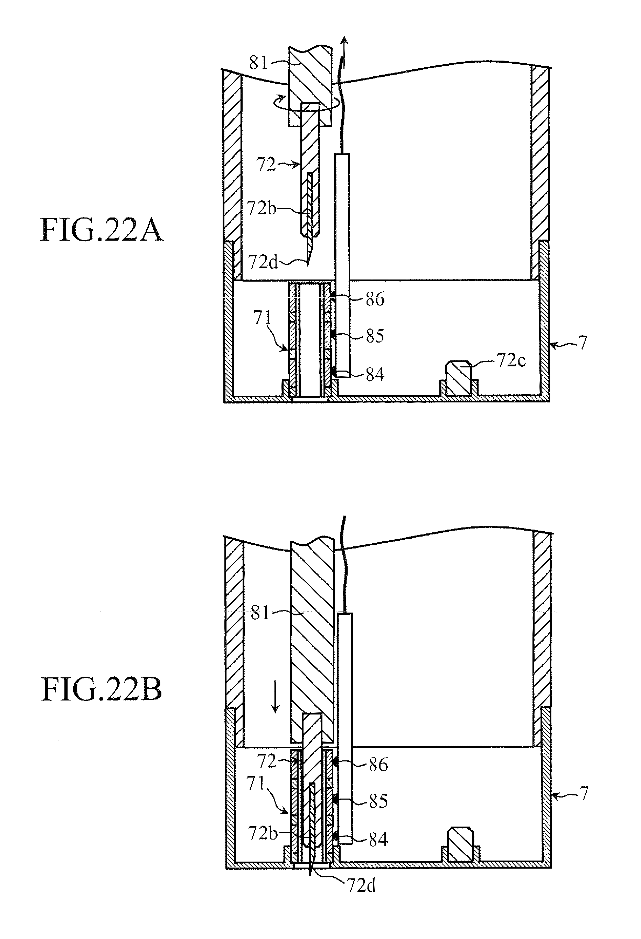

Next, the lancet holder 81 is moved upward and rotated at the same time as illustrated in Fig. 22A. Accordingly, the lancet 72 is subject to a force which wrings the cap 72c off and a force which pulls out the top section 72d of the puncture needle 72b from the cap 72c. As a result, the cap 72c has been removed and the top section 72d of the puncture needle 72b is exposed in the lancet 72.

The device 7 is rotated manually or automatically by a rotary mechanism provided in the puncture device 8, and halted when the lancet 72 is located immediately above the biosensor 71. Since the connector 82 is placed on the rotation locus of the biosensor 71 as described earlier, the contact points 84 to 86 are brought in contact with the working electrode 10, the counter electrode 11 and the detection electrode 12 in the biosensor 71.

As illustrated in Fig. 22B, the lancet holder 81 is moved downward by the drive mechanism, not shown, with the device 7 brought in contact with the part to be punctured such as skin. Then, the lancet 72 is inserted into the biosensor 71, and the puncture needle 72b is punctured through the part to be punctured. As a result, the part to be punctured, such as skin, is incised.

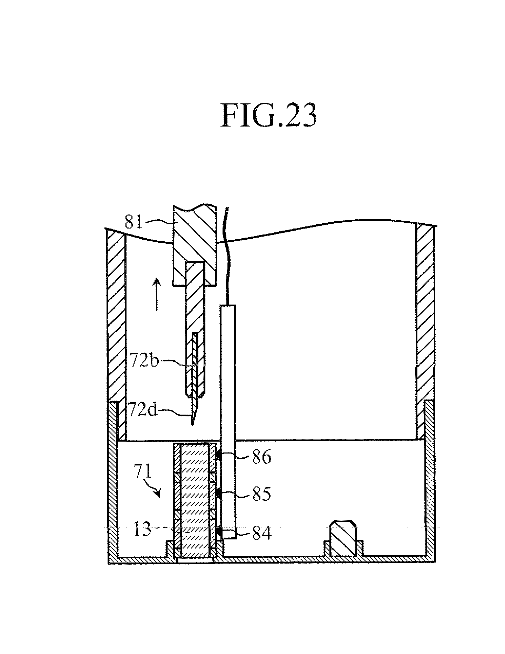

As illustrated in Fig. 23, the lancet holder 81 is moved upward so that the puncture needle 72b is pulled out from the punctured part. As a result, such a specimen as blood or interstitial fluid flows out from the punctured part and is sucked into the capillary 13 of the biosensor 71. In the capillary 13, the specimen layer 14 is dissolved by the specimen such as blood, and a liquid phase reaction system is thereby built in the capillary 13.

In the puncture device 8, it is confirmed if there is a flow of liquid junction current between the working electrode 10 (or counter electrode 11) and the detection electrode 12. Further, the puncture device 8 is subject to voltage application by way of the working electrode 10 and the counter electrode 11 so that a response current value at the time of the voltage application is measured.

In the liquid phase reaction system, oxidoreductase, for example, specifically reacts with a particular component in the specimen such as blood, and electrons are removed from the particular component. Then, the electrons are supplied to the electron transmitter, and the electron transmitter is converted to a reduced electron transmitter. When the voltage is applied to the liquid phase reaction system by means of the working electrode 10 and the counter electrode 11, the electrons are supplied to the working electrode 10 from the reduced electron transmitter. Therefore, the puncture device 8 can measure an amount of the electrons supplied to the working electrode 10 as the response current value. In the puncture device 8, a concentration of the particular component, for example, glucose, is calculated based on the response current value measured after a certain amount of time has passed since the supply of blood to the capillary 13.

## Claims

1. An analytical instrument comprising:
a plurality of electrodes formed in an annular shape with stacked on each other in an axial direction; and
a specimen layer formed in an interior of at least one of the plurality of electrodes.

2. The analytical instrument as claimed in Claim 1, wherein an insulation layer is interposed between the adjacent electrodes in the plurality of electrode.

3. The analytical instrument as claimed in Claim 2, wherein the insulation layer electrically joins the adjacent electrodes with each other.

4. The analytical instrument as claimed in Claim 1, wherein the adjacent electrodes in the plurality of electrode are coupled with each other by an insulation tube provided to mantle these electrodes with a certain space therebetween.

5. The analytical instrument as claimed in Claim 4, wherein the insulation tube is a heat-shrinkable tube.

6. The analytical instrument as claimed in Claim 1, further comprising a sealing member for embracing outer peripheral surfaces of the plurality of electrodes.

7. The analytical instrument as claimed in Claim 6, wherein the sealing member has through holes for exposing part of the outer peripheral surfaces of the plurality of electrodes.

8. The analytical instrument as claimed in Claim 1, wherein the plurality of electrodes include a working electrode and a counter electrode.

9. The analytical instrument as claimed in Claim 8, wherein the plurality of electrodes further include a detection electrode for detecting supply of a specimen to interiors of these electrodes.

10. The analytical instrument as claimed in Claim 1, wherein interiors of the plurality of electrodes are configured to exert a capillary force.

11. The analytical instrument as claimed in Claim 1, wherein an insulation layer is provided on an end surface of an outermost one in the plurality of electrodes.

12. An analytical instrument comprising:
a first electrode formed in an annular shape;
a second electrode formed in an annular shape and inserted through an interior of the first electrode with a space from an inner peripheral surface of the first electrode; and
a specimen layer formed between the inner peripheral surface of the first electrode and an outer peripheral surface of the second electrode.

13. The analytical instrument as claimed in Claim 12, wherein an end section of the second electrode projects from an end surface of the first electrode.

14. The analytical instrument as claimed in Claim 12, wherein an end section of the second electrode is retracted behind an end surface of the first electrode.

15. The analytical instrument as claimed in Claim 12, wherein an end section of the second electrode projects from an end surface of the first electrode, and another end section of the second electrode is retracted behind another end surface of the first electrode.

16. The analytical instrument as claimed in Claim 12, wherein
the first electrode and the second electrode are joined with each other by an adhesive, and
the adhesive is supplied into through holes formed in the first electrode and further adherently extended to an outer peripheral surface of the second electrode.

17. The analytical instrument as claimed in Claim 12, wherein the first electrode is a working electrode, and the second electrode is a counter electrode.

18. The analytical instrument as claimed in Claim 17, wherein the specimen layer is formed on an inner peripheral surface of the first electrode.

19. The analytical instrument as claimed in Claim 12, wherein a space between the inner peripheral surface of the first electrode and the outer peripheral surface of the second electrode is configured to exert a capillary force.

20. The analytical instrument as claimed in Claim 12, wherein at least one of an end surface and an inner surface in a lower end section of the second electrode is hydrophobized.
